# EUROPEAN PATENT APPLICATION

(11) **EP 4 621 790 A1**
(43) Date of publication of application: **24.09.2025**
(21) Application number: 24165198.3
(22) Date of filing: 21.03.2024
(51) Int. Cl.: G16H 10/60, G16H 20/00, G16H 40/40

(54) **METHOD AND SYSTEM TO TRACK PERSONNEL AND EQUIPMENT TO REASSURE A PATIENT**

(71) Applicant: Koninklijke Philips N.V., 5656 AG Eindhoven (NL)
(72) Inventor: SCHADEWALDT, Nicole, Eindhoven (NL); TASAR, Ozgur, Eindhoven (NL); NAUTS, Sanne, 5656AG Eindhoven (NL); HEUVELINK, Annerieke, Eindhoven (NL)
(74) Representative: Philips Intellectual Property & Standards

(57) **Abstract**

The present invention relates to a computer-implemented method for tracking personnel and/or equipment for reassuring a patient during a medical procedure with a medical system. The method comprises receiving an electronic representation of a medical procedure to be performed on the patient, the electronic representation comprising a sequence of steps of the medical procedure, and receiving tracking data comprising a position of medical staff performing the medical procedure or contributing to the medical procedure, and/or a position of medical equipment used in the medical procedure. The method comprises further correlating the received tracking data with the electronic representation of the medical procedure, thereby determining a current step of the sequence of steps of the medical procedure, generating patient information, i.e. information for the patient, based on the received tracking data and the determined current step of the sequence of steps of the medical procedure, and providing the generated patient information to the patient during the medical procedure.

## Description

### FIELD OF THE INVENTION

The present invention relates to a method for tracking personnel and/or equipment for reassuring a patient during a medical procedure with a medical system, a data processing apparatus for carrying out the steps of the method, and a medical system comprising the data processing apparatus.

### BACKGROUND OF THE INVENTION

During medical image acquisition or other medical procedures, the patient often has to wait, while things are happening outside his/her field of view and knowledge. These procedures can be, for example, procedures where the patient is positioned on a table of a medical device already, like magnetic resonance imaging, where coils are being fetched, the exam card is being refined, or sequences are being (re)planned, radiotherapy procedures, where the treatment device is being positioned, or the registration and clearance of the radiation plan is still being performed, or interventional procedures, where the staff is waiting for something to finalize, e.g. a medication being prepared, a sedation taking sufficient effect, a graft being created, etc. Further, any other diagnostic or interventional procedure in which staff, like an MRI technologist, a CT operator or radiotherapy-staff, needs to align with other staff like a radiologist on the imaging or radiotherapy protocol, may lead to waiting times for the patient, while the patient is on the table already. In general, any medical procedures that involve an awake patient, and where a delay due to emergencies or steps of the procedure, that might at least temporarily leave the patient unable to see or know what is happening, may occur, will leave the patient alone and wondering how long the time to wait will be. These waiting times often cannot be avoided and frequently are a normal part of the procedure.

In particular, in certain medical procedures, e.g., those that involve equipment that can harm the staff, the patient is often left alone for parts of the procedure. Typical examples are MRI scans, which might take up to one hour, and which include break times with no intervention, where the patient might wonder, what is happening around him. If nothing happens that is noticeable for the patient, many patients may get nervous and worry about the procedure or its outcome.

Waiting times during medical procedures, in particular, when the patient is already positioned on the table of the medical examination or treatment device, increase anxiety and fear of the patient, because they create space for worry, imagination about bad things that could happen, or negative outcomes of the procedure. In addition, in cases of MRI scans there is usually a critical clinical suspicion for a disease, which might also worry the patient.

Thus, the patient might wonder, what the staff does or if something is not going according to plan. This increased anxiety of the patient can compromise the smooth execution of the medical procedure or the cooperation of the patient. Currently, the patient may be informed by the staff about what is happening, and may be assured that everything is going according to the planned procedure. However, the time the staff can devote to this is limited, and when nobody is at the patient's side, there is no information at all for the patient.

The inventors of the present invention have thus found that it would be advantageous to have a method and a system that informs the patient about the current steps of the procedure, thereby reassuring the patient and leading to more relaxed patients during the procedure, that are better able to follow the instructions. This advantageously may lead to shorter workflows, less exceptions like scan aborts and/or repeats, and better results like diagnostic image quality.

### SUMMARY OF THE INVENTION

It is an object of the present invention to provide an improved method and system that informs and reassures a patient during a medical procedure about the current steps of the procedure being performed, thereby leading to a more relaxed patient that is better able to cooperate and thus improving procedural workflows and outcomes.

The object of the present invention is solved by the subject matter of the independent claims, wherein further embodiments are incorporated in the dependent claims.

The described embodiments similarly pertain to the computer-implemented method for tracking personnel and/or equipment for reassuring a patient during a medical procedure with a medical system, the data processing apparatus for carrying out the steps of the method, and the medical system comprising the data processing apparatus. The embodiments described further may be combined in any possible way. Synergistic effects may arise from different combinations in various ways of the embodiments described further although these combinations might not be described explicitly in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a (computer-implemented) method for tracking personnel and/or equipment for reassuring a patient during a medical procedure with a medical system. The method comprises receiving an electronic representation of a medical procedure to be performed on the patient, the electronic representation comprising a sequence of steps of the medical procedure, and receiving tracking data comprising a position of medical staff performing the medical procedure or contributing to the medical procedure, and/or a position of medical equipment used in the medical procedure. The method comprises further correlating the received tracking data with the electronic representation of the medical procedure, thereby determining a current step of the sequence of steps of the medical procedure, generating patient information to be provided to the patient based on the received tracking data and the determined current step of the sequence of steps of the medical procedure, and providing the generated patient information to the patient during the medical procedure.

Thus, a method for patient information is proposed based on live tracking of personnel and/or equipment. The patient is informed on a screen or via an audio system on the role and location of relevant staff and equipment. By using sensors like cameras or badges to track components of the process like equipment or personnel without intervening into the process, the patient can be kept informed in real time of what is happening. The effect of this patient information is to stop the patient worrying, by reassuring the patient about the correctness of the procedure and about the staff taking care of him. A secondary effect may be to draw the patient's attention away from the potentially negative diagnosis to the current situation, the exam or the medical procedure itself, and thus calming the patient.

The method requires as input tracking data of medical personnel and/or equipment involved in the medical procedure. These tracking data can be acquired, for example, from sensors like cameras, badges or electronic markings of equipment that are used to track involved medical personnel and equipment. Badges can include personnel badges, which may be tracked using RFID devices or similar equipment. Thus, live data of a location of the involved staff and/or the medical equipment can be received. Medical equipment could be, for example, a coil that is necessary to conduct a diagnostic imaging procedure, and that has to be appropriately placed after the patient is positioned on the table of the imaging system. Further, an electronic representation of the medical procedure needs to be available and to be received by the method. This electronic representation can be understood as a sequence of steps stored on a memory, the sequence of steps comprising the steps to be performed in the medical procedure, together with the location of the involved staff and the equipment during the respective step. Preferably, a relative position of staff and equipment with respect to each other can be stored in the electronic representation. By a comparison of the tracked position of the medical staff performing the medical imaging examination or contributing to the medical imaging examination, and/or a position of medical equipment used in a procedure of the medical imaging examination with the sequence of the steps of the electronic representation of the medical procedure, thereby correlating the received tracking data with the electronic representation, a current step of the procedure can be determined. Having determined the current step of the procedure and the state of the procedure, the next steps of the procedure can be identified. Based on the current step and the next steps, patient information is generated. Patient information is to be understood as information to be provided to the patient, and rather not in the sense of information about the patient. Information of interest to be provided to the patient is preferably about the staff, the equipment or the procedure to be performed. Preferably, the patient is provided with information that gives reassuring and calming information that the procedure is going as planned and what will happen next. This generated patient information is preferably provided to the patient via an electronic medium for communication with the patient like a screen or an audio system. By being provided with updated information on a regular basis during the medical imaging examination, the patient is reassured about the procedure and that everything is alright.

Direct effects of the proposed method are more relaxed patients during the procedure that are better able to follow instructions, which will lead to shorter workflows, with less exceptions like scan aborts and/or repeats of a scan. Additionally, better results can be achieved regarding diagnostic image quality, for example. Indirect effects can be more relaxed patients after the procedure, that are better able to cope with subsequent procedures or discussion with clinicians.

In an embodiment of the invention, the medical procedure is a medical imaging examination or an interventional procedure. Further the invention can be applied to procedures like cathlab/image-guided therapy, or radiotherapy procedures,

In an embodiment of the invention, the tracking data are acquired by analyzing a video stream of a camera-based sensor.

In an embodiment of the invention, the analyzing of the video stream is performed using a trained artificial intelligence.

In an embodiment of the invention, the tracking data are acquired by tracking a position of an electronic communication device attached to the medical staff and/or the medical equipment. In particular, one-way communication of the electronic communication device may be preferred, specifically magnetic cards or labels positioned on or at equipment or the personnel, that can be read out by a reading device.

In an embodiment of the invention, the patient information is provided to the patient via a screen and/or an audio system.

In an embodiment of the invention, the method further comprises receiving examination data from the medical procedure comprising a state of the medical procedure, the state of the medical procedure being correlated to the sequence of steps of the medical procedure. Thus, for example, information can be provided from the medical system whether a scan is currently being performed. Alternatively, for example, a microphone can be used to detect noise in a scanner room of an imaging system, which can be used to decide whether a scan is currently performed. This may be particularly useful if the imaging system is a magnetic resonance imaging system MRI.

In a minimal implementation no direct connection, so information, from the active medical equipment, like an MRI-scanner, a CT-scanner, or a patient monitoring device, is necessary, as all information about the procedure and the workflow, incl. patient and staff status, can be deduced indirectly through sensors like cameras. However, information about what is happening, including at the moments of interest when the patient is waiting, like during a pause between MRI scans, may in an extended embodiment be directly gathered from the status of the system. For example, in case of the MRI scanner, it can be directly deduced from the scanner whether a table is moving in or out of the bore, if a scan is happening, whether a staff member is planning the exam, or whether images are being checked, etc.

In an embodiment of the invention, the patient information is continuously updated during the medical procedure based on the received tracking data and the determined current step of the sequence of steps.

In an embodiment of the invention, the patient information is provided to the patient in case an interruption of the medical procedure is determined in which the medical system is inactive for a period of time exceeding a predefined threshold.

In an embodiment of the invention, the patient information is generated based on information about the medical staff, the medical equipment, and/or the medical procedure received from a database.

In an embodiment of the invention, the patient information comprises personal and/or professional information regarding the staff, a location of the staff, information about the medical equipment, a location of the medical equipment, and/or information about the current step and/or a subsequent step of the medical procedure.

In an embodiment of the invention, the generated patient information is provided to the patient such that the patient is comforted and reassured with respect to the medical procedure, thereby ensuring a smooth medical procedure providing high-quality results of the procedure like medical images.

According to another aspect of the invention, there is provided a data processing apparatus for carrying out the steps of the method according to any one of the preceding embodiments.

According to another aspect of the invention, there is provided a medical system comprising the data processing apparatus of any of the preceding embodiments, a tracking system configured for acquiring tracking data comprising a position of medical staff performing a medical procedure or contributing to the medical procedure, and/or a position of medical equipment used in the medical procedure, and a patient interface configured for providing generated patient information to a patient during a medical procedure performed with the medical system.

According to another aspect of the invention, there is provided a computer program comprising instructions which, when the program is executed by a computer, cause the computer to carry out the steps of the method according to any one of the preceding embodiments.

According to another aspect of the invention, there is provided a computer-readable storage medium comprising instructions which, when executed by a computer, cause the computer to carry out the steps of the method according to any one of the preceding embodiments.

Thus, the benefits provided by any of the above aspects equally apply to all of the other aspects and vice versa.

In summary, the invention relates to a computer-implemented method for tracking personnel and/or equipment for reassuring a patient during a medical procedure with a medical system. The method comprises receiving an electronic representation of a medical procedure to be performed on the patient, the electronic representation comprising a sequence of steps of the medical procedure, and receiving tracking data comprising a position of medical staff performing the medical procedure or contributing to the medical procedure, and/or a position of medical equipment used in the medical procedure. The method comprises further correlating the received tracking data with the electronic representation of the medical procedure, thereby determining a current step of the sequence of steps of the medical procedure, generating patient information to be provided to the patient based on the received tracking data and the determined current step of the sequence of steps of the medical procedure, and providing the generated patient information to the patient during the medical procedure.

One of the advantages of embodiments of the present invention is that the patient is reassured about the procedure and about the staff taking care of him, thereby stopping worries. A secondary effect may be that the patient's attention is drawn away from the potentially negative diagnosis to the current situation, the exam or medical procedure itself, and thus being calmed. Therefore, direct effects are more relaxed patients during the procedure, that are better able to follow instruction, which may lead to shorter workflows with fewer workflow interruptions. For example, unnecessary scan aborts and/or repeats of the scan induced by the patient can be avoided. Thus, better results regarding diagnostic image quality can be advantageously achieved. Indirect effects may be more relaxed patients after the procedure, that are better able to cope with subsequent procedures or discussion with clinicians.

The problem is solved by a patient information system and method based on the live tracking of personnel and equipment, which informs the patient on a screen or via audio systems on the role and location of relevant staff and/or equipment during the procedure.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a block diagram of a computer-implemented method for tracking personnel and/or equipment for reassuring a patient during a medical procedure with a medical system according to an embodiment of the invention.
Fig. 2 shows a schematic setup of a medical system comprising a data processing apparatus for performing the method according to the invention, a tracking system, and a patient interface.
Fig. 3 shows an example of a reasoning engine to control the display of patient information to a patient.
Figs. 4A and 4B show two examples of patient information generated according to the method of the invention and provided to a patient during a medical procedure.

### DETAILED DESCRIPTION OF EMBODIMENTS

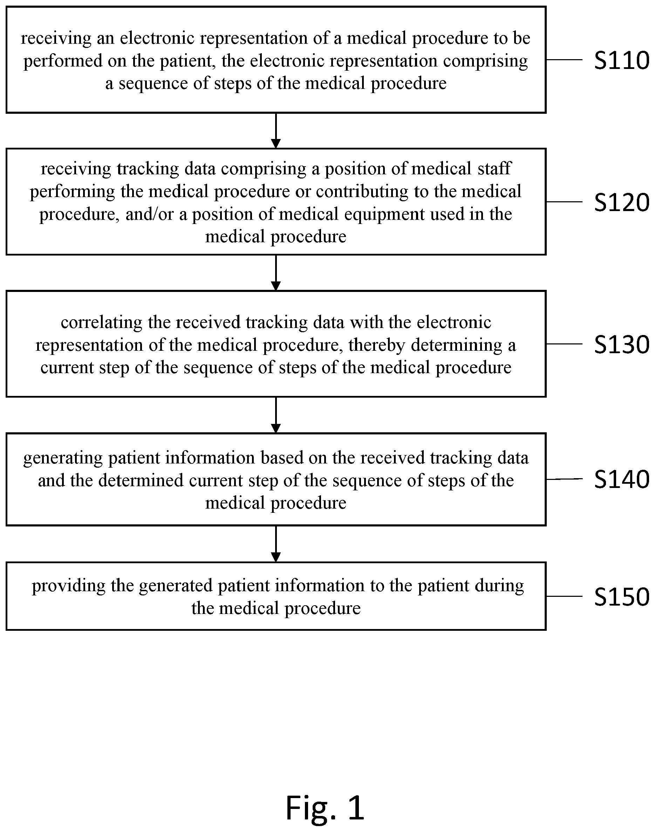

Fig. 1 shows a block diagram of a computer-implemented method for tracking personnel and/or equipment for reassuring a patient 110 during a medical procedure with a medical system according to an embodiment of the invention. The method comprises the step S110 of receiving an electronic representation 140 of a medical procedure to be performed on the patient 110, the electronic representation comprising a sequence of steps of the medical procedure, and the step S 120 of receiving tracking data 150 comprising a position of medical staff 120 performing the medical procedure or contributing to the medical procedure, and/or a position of medical equipment 130 used in the medical procedure. The method comprises further the step S130 of correlating the received tracking data 150 with the electronic representation 140 of the medical procedure, thereby determining a current step of the sequence of steps of the medical procedure, the step S140 of generating patient information 160 based on the received tracking data 150 and the determined current step of the sequence of steps of the medical procedure, and the step S150 of providing the generated patient information 160 to the patient 110 during the medical procedure.

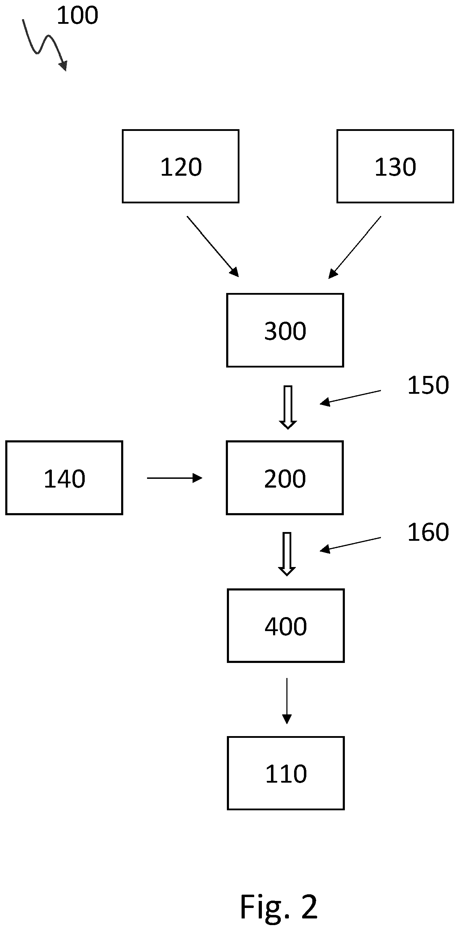

Fig. 2 shows a schematic setup of a medical system 100 comprising a data processing apparatus 200 for performing the method according to the invention, a tracking system 300, and a patient interface 400. An example of a medical procedure can be the acquisition of an MR image of a patient 110, with an MRI system as medical imaging system 100. The patient 110 is reassured during the procedure by providing him with patient information 160 generated according to tracking data 150 received by tracking personnel like medical staff 120 and/or medical equipment 130.

For tracking the medial staff 120 and the medical equipment 130, the tracking system 300 is preferably provided with one or more cameras present in the room or rooms in which the procedure is being performed. Preferably, a video stream received from one or more cameras is analyzed, and the analyzing is preferably performed using a trained artificial intelligence. In addition or alternatively, tracking can be performed by electronically measuring the position of badges, cards, or transponders attached to the medical staff 120 or the medical equipment 130 to track personnel and coils, for example, as well as other accessories. Further, the patient can be tracked, and a microphone can be provided to detect MR noise, for example.

Preferably, cameras can be present in an examination room, to observe the patient and to be able to detect issues quickly and to intervene, if necessary. Further, cameras can be mounted on or in relation to medical machinery, to aid or automate patient positioning or procedure execution. In addition or alternatively, cameras on displays present in the workflow can be used, e.g. a wall display or a computer needed to operate medical machinery, or cameras that can be mounted in the hallway or operating rooms for security reasons, as well as cameras mounted in examination rooms and operating rooms for specifically that purpose. Furthermore, electronic chips, RFID tags, or other sensing technology present in badges of personnel, can be tracked, a well as electronic chips on equipment like MR coils for tracking or security reasons, e.g. to assure, that the right coil is used and that it is registered automatically, when positioned on the MR table. However, preferably, cameras and/or electronic chips are used for the method according to the invention that are specifically installed for that purpose. Also, other sensing technologies can be used that can be present in equipment in the MR room, including mobile phones, 'smart' LEDs, etc., including LIDAR, camera-based sensors, infrared sensors, ultrasonic sensors, voice sensors, microwave sensors, electromagnetic sensors, or motion sensors, etc. Having tracked the position of the medical staff 120 and the medical equipment 130, with the tracking system 300, these tracking data 150 are provided to a data processing apparatus 200.

This data processing apparatus 200 also receives an electronic representation 140 of a medical imaging examination to be performed on the patient 110, which comprises a sequence of steps of the medical imaging examination to be performed on the patient. It may be challenging to keep the electronic representations 140 of procedures sufficiently simple to avoid constant need for adaptation and sufficiently complex to accommodate most possible procedure differences. However, for the purpose of the proposed invention it may be sufficient as a minimal implementation, to define a starting time, an end time, and pause-times, where patient information available could be presented to the patient.

Taking this example of an MR image acquisition procedure, the definition of the electronic representation 140 could be as follows:
- Start time: camera detects patient on the table and nurse leaves the room for the first time,
- End time: camera detects patient getting up from the table, and
- Pause times: camera detects no staff in the vicinity of the patient, no table movement, and uniform or no sound from the MR for longer than, for example, 30 seconds.

Any more elaborate representation of the procedure is possible, but may be not necessarily required.

An example of a more elaborate electronic representation 140 of the procedure is given in the following:
a. patient setup (position patient on table) by staff in MR room
b. fetch and position coil (and other accessories) by staff in MR room
c. move patient into the bore of the scanner by staff in MR room
d. execute exam (detailed info could be retrieved from exam card) by staff at MR console
   i. execute initial survey scan
   ii. plan upcoming diagnostic sequences
   iii. execute diagnostic scans
   iv. final check whether all OK / required information is in
e. move patient out of the bore of the scanner by staff in MR room
f. remove coil (and other accessories) by staff in MR room.

Together with this sequence of steps, the electronic representation 140 comprises preferably the current position of the medical staff 120 and the necessary medical equipment 130, for example, whether they are at the console, in the MR room, at the patient, etc.

Having received the tracking data 150 and the electronic representation 140, the data processing apparatus 200 correlates the received tracking data 150 with the electronic representation 140 of the medical imaging procedure, and thereby determines a current step of the sequence of steps of the medical imaging examination, and generates patient information 160 based on the received tracking data 150 and the determined current step of the sequence of steps of the medical imaging examination.

This patient information 160 is provided to the patient 110 via a patient interface 400. A screen with optional audio connection can be available to communicate with the patient 110, for example. There may be different options for the patient interface possible. Preferably, there is an audiovisual communication with the patient available. Like an ambient experience system, a screen or a projection can be present outside the bore or in bore, plus an audio connection available over the system. However, any other means of visualization like a screen or projection or goggles could be used for this purpose. If no visual means of communication with the patient is available, an audio connection can also be used. Audio may be available within any system, even without a screen, as audio-connection is also available in any current MR system to communicate with the patient on an as-needed-basis. If the procedure is MR image acquisition, audio-communication with the patient might not be preferred while the MR is producing loud noise, so there may be situations, where the audio communication may not be preferred, and video communication only may be used. Alternatively, other means of communication could be used, e.g., touch systems for blind people to communicate haptically what in other cases may be said or displayed.

Based on the information in a database, communication of information to the patient can be triggered which can be displayed on screen or communicated via audio. This information may include the following, prior saved details: For clinical personnel: Name, title, photograph, and professional information like years of experience, training, special qualifications, or for medical equipment like an MR coil: name, photograph, and typical use information. The information may also include the following live details: Location of personnel or equipment, e.g. the room, the position of staff or equipment: e.g., coil on couch, or personnel in front of coil cabinet. Further, actions of personnel, e.g. fetching coil, operating MR exam, talking to colleague, distances of personnel to the patient, or a state of the exam can be communicated, e.g., "MR exam is being prepared" as long as checkmarks like "coil positioned" are still open.

Communication with the patient could look as follows: As soon, as the patient is on the table and the personnel not with the patient anymore, the system can start displaying information on procedure progress, of course in any case carefully phrased to re-assure and not to make the patient insecure. If the coil is not on the patient and the personnel is fetching the coil, the screen can show the staff member fetching the coil and the coil being used for this exam and provide additional information, e.g. on the reason for using this coil, or a photograph of how this coil is going to be positioned. If the patient is positioned on the table with the coil, but the table does not yet move into the bore, the screen and audio can display information about the staff member to surpass the waiting time, e.g., re-assuring information on the staff members expertise on this job, the distance of the staff member to the patient, e.g. "just 2 meters away in the MR operating room", or information about other staff members involved in the process and their position or role, e.g. preparing the exam at the MR operation table. If the patient is within the bore, a first scan was done and there is a pause before the next scan, information can be displayed on how an MRI exam always starts with an overview image (`like a map') to plan the course of action. If the patient is within the bore, a diagnostic (so not first) scan was done and there is a pause before the next scan, information can be displayed on how each scan delivers an image that needs checking to see whether it was successful ("sharp, the region of interest in focus"). Alternatively, simple information can be shown about what is happening, e.g., "There is a brief pause in the scan. Do not worry, this is a perfectly normal part of an MRI-the scan will continue shortly". If position change of personnel is detected, e.g. the staff member at the MR operating table discussing with some other staff member, it can be displayed to the patient, who is discussing with whom, and re-assuring information on those staff members can be displayed.

With this information, the patient is reassured about the procedure and about the staff taking care of him, thereby stopping worries. Thus, the patient's attention is drawn away from the potentially negative diagnosis to the current situation, the exam or medical procedure itself, and thus being calmed. Therefore, the patients can be more relaxed during the procedure, and better able to follow instruction, which may lead to shorter workflows with less exceptions. For example, unnecessary scan aborts and/or repeats of the scan induced by the patient can be avoided. Thus, better results regarding diagnostic image quality can be advantageously achieved. Also, the patients can be more relaxed after the procedure, and thus better able to cope with subsequent procedures or discussion with clinicians.

There are different options, how the patient information can be presented to the patient, which may have different soothing effects. Also, there are different options to animate the information display. For example, the background of the display can be faded, while patient information is displayed, the patient information can be faded in as one block, or line-by-line. Some part of the text indicating the next action can be highlighted, or there can be transition options from a theme display to an information display.

The communication with the patient may be designed as script-based information panels with added visuals to be superimposed on a screen. However, this could be much more generic and interactive. As one embodiment, an AI based avatar could communicate the information to the patient, still using photographs and script for support, but engaging with the patient, creating a "story" and connecting items, and maybe adapting to the patient information needs. This avatar could also be taken from other information procedures beyond the medical procedure at hand, e.g. it could be the same avatar that was used for preparatory content given to the patient previously.

Another example may be the acquisition of CT images for oncology. Although the image acquisition itself is much shorter than MR, the preparation might take quite long to position the patient optimally for tumor imaging, or to prepare contrast or similar. Also, the staff will be with the patient for a longer part of the total procedure, so if they should leave the room and the procedure does not start immediately the patient might get more anxious. Although the detailed description focuses rather on the MR example, of course the principles can be applied to other procedures as well.

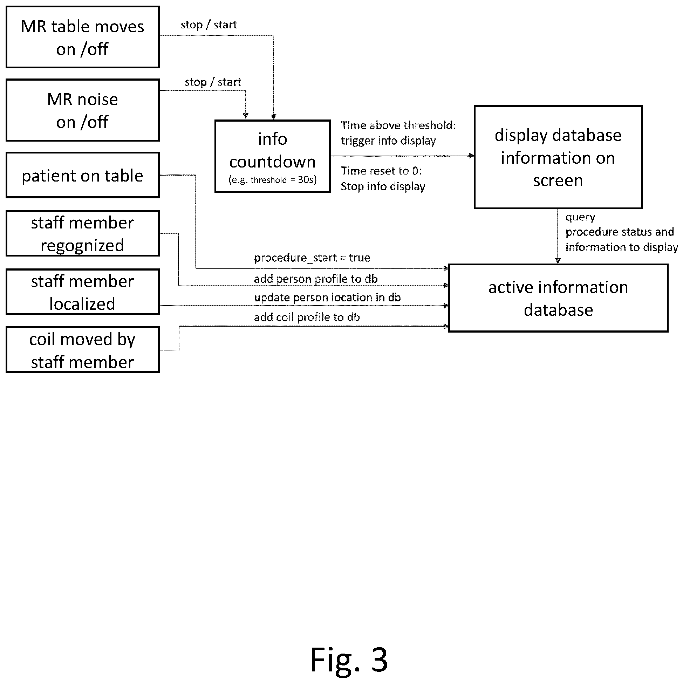

Fig. 3 shows an example of a reasoning engine to control the display of patient information 160 to a patient 110. This may be part of a data processing apparatus executing a program having one or more of the following input data:
- A camera video stream from available cameras with additional context information on the cameras, e.g. which people can be observed by a camera, e.g. if the patient is expected in view of the camera, and at what step in the procedure,
- Audio input data from microphones in the examination room, or other microphones,
- Tracking data from tagged equipment or personnel, for example from badges, or
- Additional information for equipment or personnel to track, e.g. pictures of staff and MR coils, potentially at angles at which they are expected to appear in the camera. An algorithm that may have been trained using training input data of the above type, is able in real-time to recognize, localize and track personnel and equipment. Further, the algorithm may have a reasoning engine to determine from any combination of personnel and equipment localizations a state, which might trigger an action. Below is a description of possible states of this reasoning engine shown in Fig. 3.

The following are examples of possible states:
- A patient enters the room, recognizable by not wearing medical gowns: start tracking the patient
- A member of medical staff is visible in the examination room: identify member of medical staff (e.g. compare to database picture, localize badge), set this member of staff the "patient responsible" for the current exam, add basic information of this staff member to "current information database"
- Patient is positioned on the MR table, no staff is visible at a distance below a threshold Action: Start "info countdown"
- "info countdown" hits 30s: display data from "current information database" on screen
- Table motion detected: stop "info countdown", stop "display data", set "table moving = on, observe"
- While observe table, table motion stops: set "table moving = off", if "MR exam = off": Start "info countdown"
- MR noise detected: stop "info-countdown", stop "display data", set "MR exam = on, observe"
- While observe MR noise, if MR noise below threshold: set "MR exam = off', if "table moving = off': start "info-countdown"
- Personnel is visible on MR operating room camera/camera in the hallway/examination room camera, but away from the patient: identify member of medical staff; if this member of medical staff is "patient responsible", add location information including distance to patient to "current information database"; if this member of staff is not "patient responsible": add to "current information database", but only display, if in contact with "patient responsible" staff.
- Coil is visible on camera close to medical personnel: observe if this coil is being moved more than a certain threshold / away from storage position: add basic coil information and coil location information to "current information database".
- Tracked coil is installed on MR table: update coil location information in "current information database".

Many more scenarios can be considered and added, however, this already gives an example, how such an algorithm to determine the state of the procedure can be programmed.

Thus, based on the patient position on the table, the algorithm can deduce that the patient setup is completed. Based on the coil position, the algorithm can deduce if the step of the coil positioning is already completed. Based on the table position (inside or outside bore), the algorithm can deduce, if the patient is already moved into the bore, thereby completing the respective step. Based on the personnel position (in MR room or outside at MR console) with respect to the MR, the algorithm can deduce, if the exam is about to start. If MR noises are heard, the algorithm can deduce that scanning takes place. If for the first time after MR noises were heard there is a silence, the algorithm can deduce that the first (survey) scan took place. If a staff member interacts with the console after the first scan while no MR noises are audible, it can deduce that the further exam is being planned. If multiple staff members are present and in conversation, e.g. facing each other, and there is a longer pause of MR noise, the system can deduce that there is a halt to the examination and it is being discussed, if e.g. updates to the planned sequences are required, or the diagnostic quality of the scans is sufficient.

There are multiple options to produce the patient information to be displayed to the patient. These include, but are not limited to:
- Pre-phrased information on medical staff, equipment, and certain procedure steps, phrased carefully to re-assure and not trigger un-easiness.
- A database of information on staff, equipment and procedure steps, where the display is configurable. The simplest form may be to provide careful formulations proven to re-assure to communicate certain properties or procedure steps. Also, there could be filters for experience, showing the years of experience of the staff only if above a certain threshold, or that junior staff members are not displayed, if there is no compelling trustable information for experience available.
- Based on a database, there could also be filters to display the information in different ways suitable for the patient, e.g. as pictograms and simplified videos or graphics for young children, with simplified language for children, or with different levels of depth depending on the patient's information needs. Additional filters for personalization are possible, e.g. considering gender, or other properties of the patient, which might lead to different selection of database properties, different formulation of the same properties, or different display. The patient properties necessary for patient-based customization of display could be inserted by the staff, by the patient or deduced from available medical records.
- Additionally, the actual status of the patient could be monitored and used to control the displaying of information. For example, when it is noticed that a patient is asleep and if that is not a problem, the audiovisual communication may be paused. Or, when it is deduced that a patient is very anxious, the most reassuring, comforting information can be displayed, while attentive readers of factual information may trigger the provision of even more in-depth information.

While it may be hard coded what and who should be tracked, specifically the personnel and the coils, the database of trackable items can also be created and trained dynamically by an artificial intelligence engine. An AI engine can be initialized with information on certain items. For example, pictures from different angles of the personnel or equipment, plus background information, pictures from personnel badges, including personnel numbers or other information. Further, equipment can be used for training, which does not need to be presented to the patient but is indicative of a procedure step, like rolled up towels to position the patient, or head masks for radiotherapy fixation. In the training phase, the AI engine needs information on the procedure step and confirmation, if an item is detected correctly or not. Then, the engine will learn to recognize items better, to relate item detection at certain positions to procedure steps, and to identify additional items that can be used to identify or confirm procedure steps. In the application phase, the AI engine is able to identify the items and procedure steps much more reliably and very specific for that site, as supportive items may differ significantly between sites. In this case, the information display to the patient would only show items with background information, supportive items like rolled up towels without background information would be omitted and serve to identify procedure steps only.

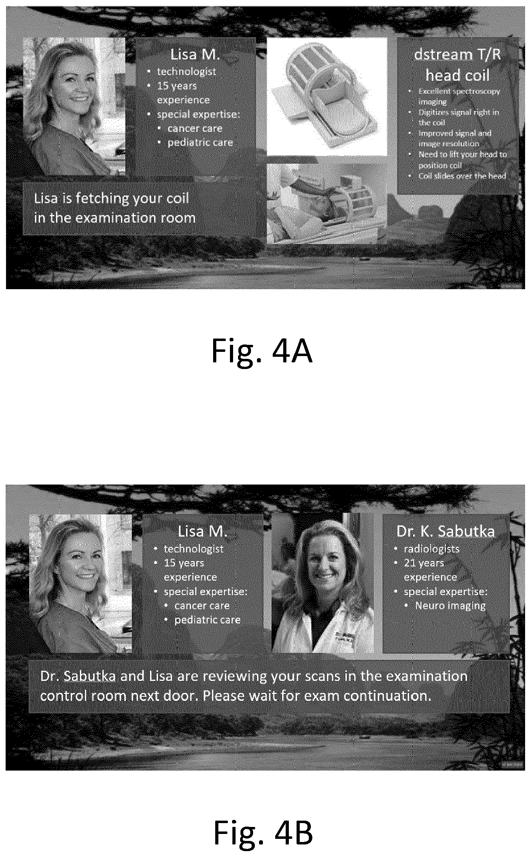

Figs. 4A and 4B show two examples of patient information 160 generated according to the method of the invention and provided to a patient 110 during a medical procedure, including information on personnel, equipment and status of procedure superimposed on ambient experience theme. At the start of the examination, the patient is already on the table, but technologist Lisa is still fetching the coil. To bridge the time it takes to get the coil, information about Lisa and about the coil and how it is going to be positioned are displayed to the patient, which is shown in Fig. 4A. In Fig. 4B, the MR examination has come to a longer pause and the patient is not aware, what is going on. However, the tracking system has identified radiologist Dr. Sabutka to stand in front of the MR control panel in the control room. Whatever Lisa and Dr. Sabutka are discussing as long as the procedure is not continuing may not be known, however, it is assumed that they are discussing the preliminary scan results and the patient is thus informed that people close to him are available and are keeping watch over him. Further examples could be that a technician is adjusting the MR exam between scans, e.g. adding a new scan, because a scheduled scan was unsuccessful, or that the technician is coming to the patient as the examination is over.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

It is to be understood that any of the previous steps described in relation to embodiments and/or training steps described above can be performed by a specific-purpose computer system or general-purpose computer system, or a computer-readable medium, or data carrier system configured to carry out any of the steps described previously. The computer system can include a set of software instructions that can be executed to cause the computer system to perform any of the methods or computer-based functions disclosed herein. The computer system may operate as a standalone device or may be connected, for example using a network, to other computer systems or peripheral devices. In embodiments, a computer system performs logical processing based on digital signals received via an analogue-to-digital converter.

Some portions of the description are presented in terms of symbolic representations of operations on non-transient signals stored within a computer memory. These descriptions and representations are used by those skilled in the data processing arts to convey the substance of their work most effectively to others skilled in the art. Such operations typically require physical manipulations of physical quantities. Usually, though not necessarily, these quantities take the form of electrical, magnetic, or optical signals capable of being stored, transferred, combined, compared, and otherwise manipulated. It is convenient at times, principally for reasons of common usage, to refer to these signals as bits, values, elements, symbols, characters, terms, numbers, or the like. Furthermore, it is also convenient at times to refer to certain arrangements of steps requiring physical manipulation of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage devices. Portions of the present disclosure include processes and instructions that may be embodied in software, firmware, or hardware, and when embodied in software, may be downloaded to reside on and be operated from different platforms used by a variety of operating systems.

In a networked deployment, the computer system operates in the capacity of a server, or as a client user computer in a server-client user network environment, or as a peer computer system in a peer-to-peer or distributed network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions sequentially or non-sequentially that specify actions to be taken by that machine. The computer system can be incorporated as an integrated system part of a larger system that includes additional devices. In an embodiment, the computer system can be implemented using electronic devices that provide voice, video, or data communication possibilities. Further, while the computer system is illustrated in the singular, the term "system" shall also be taken to include any collection of systems or sub-systems that individually or jointly execute a set or multiple sets, of software instructions to perform one or more computer functions.

The computer system may also include a processor. The processor executes instructions to implement some, or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device, a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices. The processor can include one or more internal levels of cache, and a bus controller or bus interface unit to direct interaction with a bus. The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection, or network, of computing devices each including a processor or processors. Programs have software instructions performed by one or multiple processors that may be within the same computing device or which may be distributed across multiple computing devices. Further, the software instructions, when executed by the processor, perform one or more steps of the methods and processes as described herein.

The computer system further includes a main memory and a static memory, where memories in the computer system communicate with each other and the processor via a bus. Either or both main memory and the static memory may be considered representative examples of the memory of the controller, and store instructions used to implement some, or all aspects of methods and processes described herein. Memories described herein are tangible storage mediums for storing data and executable software instructions and are non-transitory during the time software instructions are stored therein. The main memory and the static memory are articles of manufacture and/or machine components. The main memory and the static memory are computer-readable mediums from which data and executable software instructions can be read by a computer (or e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM (Compact Disk - Read Only Memory)), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices. The memory may store various software applications including computer executable instructions, that when run on the processor, implement the methods and systems set out herein. Other forms of memory, such as a storage device and a mass storage device, may also be included and accessible by the processor (or processors) via the bus. The storage device and mass storage device can each contain any or all of the methods and systems discussed herein.

The computer system can further include a communications interface by way of which the computer system can connect to networks and receive data useful in executing the methods and system set out herein as well as transmitting information to other devices. The computer system further includes a video display unit as an output device by which information can be output, such as a liquid crystal display (LCD), an organic light emitting diode (OLED), a flat panel display, a solid-state display, or a cathode ray tube (CRT), for example. Additionally, the computer system includes an input device, such as a keyboard/virtual keyboard or touch-sensitive input screen or speech input with speech recognition, and a cursor control device, such as a mouse or touch-sensitive input screen or pad. The computer system also optionally includes a disk drive unit, a signal generation device, such as a speaker or remote control, and/or a network interface device.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any programming language that is sufficient for achieving the techniques and implementations of the present disclosure may be used. In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to fully describe all the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the Figures are to be regarded as illustrative rather than restrictive.

Although specific embodiments have been illustrated and described herein, it should be appreciated that any subsequent arrangement designed to achieve the same or similar purpose may be substituted for the specific embodiments shown. This disclosure is intended to cover all subsequent adaptations or variations of various embodiments. Combinations of the above embodiments, and other embodiments not specifically described herein, will be apparent to those of skill in the art.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

### LIST OF REFERENCE SIGNS:

- 100: medical system
- 110: patient
- 120: medical staff
- 130: medical equipment
- 140: electronic representation
- 150: tracking data
- 160: patient information
- 200: data processing apparatus
- 300: tracking system
- 400: patient interface

## Claims

1. A computer-implemented method for tracking personnel and/or equipment for reassuring a patient (110) during a medical procedure performed on the patient with a medical system (100), the method comprising:
receiving (S110) an electronic representation (140) of a medical procedure to be performed on the patient (110), the electronic representation comprising a sequence of steps of the medical procedure;
receiving (S120) tracking data (150) comprising a position of medical staff (120) performing the medical procedure or contributing to the medical procedure, and/or a position of medical equipment (130) used in the medical procedure;
correlating (S130) the received tracking data (150) with the electronic representation (140) of the medical procedure, thereby determining a current step of the sequence of steps of the medical procedure;
generating (S140) patient information (160) to be provided to the patient based on the received tracking data (150) and the determined current step of the sequence of steps of the medical procedure; and
providing (S150) the generated patient information (160) to the patient (110) during the medical procedure.

2. The method according to claim 1, wherein the tracking data (150) are acquired by analyzing a video stream of a camera-based sensor.

3. The method according to claim 2, wherein the analyzing of the video stream is performed using a trained artificial intelligence.

4. The method according to any one of the preceding claims, wherein the tracking data (150) are acquired by tracking a position of an electronic communication device attached to the medical staff (120) and/or the medical equipment (130).

5. The method according to any one of the preceding claims, wherein the patient information (160) is provided to the patient (110) via a screen and/or an audio system.

6. The method according to any of the preceding claims, further comprising
receiving examination data from the medical procedure comprising a state of the medical procedure, the state of the medical procedure being correlated to the sequence of steps of the medical procedure.

7. The method according to any one of the preceding claims, wherein the patient information (160) is continuously updated during the medical procedure based on the received tracking data (150) and the determined current step of the sequence of steps.

8. The method according to any one of the preceding claims, wherein the patient information (160) is provided to the patient (110) in case an interruption of the medical procedure is determined in which the medical system (110) is inactive for a period of time exceeding a predefined threshold.

9. The method according to any one of the preceding claims, wherein the patient information (160) is generated based on information about the medical staff (120), the medical equipment (130), and/or the medical procedure received from a database.

10. The method according to any one of the preceding claims, wherein the patient information (160) comprises personal and/or professional information regarding the medical staff (120), a location of the medical staff (120), information about the medical equipment (130), a location of the medical equipment (130), and/or information about the current step and/or a subsequent step of the medical procedure.

11. The method according to any one of the preceding claims, wherein the generated patient information (160) is provided to the patient (110) such that the patient (110) is comforted and reassured with respect to the medical procedure, thereby ensuring a smooth medical procedure providing high-quality results.

12. A data processing apparatus (220) for carrying out the steps of the method according to any one of claims 1 to 11.

13. A medical system (100) comprising
the data processing apparatus (200) of claim 12;
a tracking system (300) configured for acquiring tracking data (150) comprising a position of medical staff (120) performing a medical procedure or contributing to the medical procedure, and/or a position of medical equipment (130) used in the medical procedure; and
a patient interface (400) configured for providing generated patient information (160) to a patient (110) during a medical procedure performed with the medical system (100).

14. A computer program comprising instructions which, when the program is executed by a computer, cause the computer to carry out the steps of the method according to any one of claims 1 to 11.

15. A computer-readable storage medium comprising instructions which, when executed by a computer, cause the computer to carry out the steps of the method according to any of claims 1 to 11.
